# EUROPEAN PATENT APPLICATION

(11) **EP 3 289 967 A2**
(43) Date of publication of application: **07.03.2018**
(21) Application number: 17175462.5
(22) Date of filing: 12.06.2017
(51) Int. Cl.: A61B 5/00, A61B 5/053, A61B 5/024, A61B 5/103

(54) **HEALTH BAND TERMINAL**

(30) Priority: 04.09.2016 US 201662383481 P; 23.09.2016 KR 20160122327
(71) Applicant: LG Electronics Inc., Yeongdeungpo-Gu Seoul 07336 (KR)
(72) Inventor: SHIM, Hongjo, 06772 Seoul (KR); PARK, Mihyun, 06772 Seoul (KR); LEE, Hyunok, 06772 Seoul (KR)
(74) Representative: Frenkel, Matthias Alexander

(57) **Abstract**

A health band terminal comprises a main body installable in one area of a body of a user; a first electrode portion arranged on one surface of the main body, having first and second electrodes arranged to adjoin each other; a second electrode portion arranged on the other surface of the main body; and a controller for calculating body component information by using an impedance value measured by the first and second electrode portions.

## Description

### BACKGROUND OF THE INVENTION

### 1. Field of the Invention

The present invention relates to a health band terminal worn on a wrist, comprising a sensing module for collecting body component information.

### 2. Background of the Invention

A mobile terminal includes all devices provided with a battery and a display unit, outputting information to the display unit by using a power source supplied from the battery and formed to allow a user to carry them. The mobile terminal includes a device for recording and playing moving pictures and a device for displaying a graphic user interface (GUI), and includes notebook computer, cellular phone, and glasses, watch, game machine, etc., which can display screen information.

Recently, a function of collecting body information through a sensor provided in a wearable device worn on a body of a user has been studied. However, since this sensor should be provided outside the device to be in contact with a body of a user, esthetic appearance is reduced. Also, while the sensor is being in contact with the body of the user, it is difficult to maintain a pose suitable to collect body component information, whereby it is difficult to provide an exact measurement result.

### SUMMARY OF THE INVENTION

Therefore, an object of the present invention is to substantially obviate one or more problems due to limitations and disadvantages of the related art.

Another object of the present invention is to provide a health band terminal for improving esthetic appearance of a health band device while taking a pose suitable for measurement of a body component.

To achieve these and other advantages and in accordance with the purpose of the invention, as embodied and broadly described herein, a health band terminal according to one embodiment of the present invention comprises a main body installable in one area of a body of a user; a first electrode portion arranged on one surface of the main body, having first and second electrodes arranged to adjoin each other; a second electrode portion arranged on the other surface of the main body; and a controller for calculating body component information by using an impedance value measured by the first and second electrode portions, wherein the second electrode portion is protruded from the other surface of the main body to touch the body of the user if the main body is installed in the body of the user.

As an example according to the present invention, the second electrode portion is arranged to adjoin a PPG sensor unit that includes a light receiving sensor and at least one light emitting diode, and the PPG sensor unit is formed on a first protrusion protruded from the other surface of the main body at a predetermined thickness. Since the second electrode portion is formed on the first protrusion, body component measurement quality may be improved by tightly adhering the electrode to the body of the user without deterioration of quality of the PPG sensor unit.

As an example according to the present invention, the health band terminal further comprises a display unit arranged on the one surface of the main body to adjoin the first electrode portion, sensing a touch input, and the controller controls the display unit to output guide information for guiding a position of a finger, which touches the first electrode portion. The display unit outputs a warning image if a touch is sensed in an area other than a predetermined area on the display unit in a state that the finger of the user touches the electrode portion, thereby guiding the user to exactly touch the electrode portion with the finger.

As an example according to the present invention, since the controller may form different control commands based on that the finger of the user touches at least one of the first and second electrodes of the first electrode portion, a component such as a push key may be omitted.

As an example according to the present invention, the health band terminal further comprises a PPG sensor unit installed in the main body, including a light receiving sensor and a light emitting diode, senses a skin color based on intensity of light received by the light receiving sensor, and the controller calculates the body component information by using a body fat algorithm based on the skin color. Therefore, the body component information may be acquired exactly by an algorithm suitable for a race of the user in accordance with the skin color of the user.

According to the present invention, since the first electrode portion includes first and second electrodes arranged to adjoin each other, the body of the user may touch the first electrode portion by using one finger. Therefore, since it is easy for the user to take a proper pose for body component measurement, exact measurement data may be acquired.

Also, since different control commands may be formed based on that the finger touches the first and second electrodes, the user input unit comprised as a push key may be omitted. Therefore, a band terminal favorable for waterproof, having esthetic appearance may be implemented.

Also, based on a touch range of a touch input applied to the display unit, a touch to the first and second electrodes may be guided or an output voltage value may be adjusted, whereby a measurement value may be acquired more exactly.

Also, body component data may be calculated by application of different algorithms in accordance with the skin color of the user.

Further scope of applicability of the present application will become more apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from the detailed description.

### BRIEF DESCRIPTION OF THE DRAWING

The accompanying drawings, which are included to provide a further understanding of the invention and are incorporated in and constitute a part of this specification, illustrate exemplary embodiments and together with the description serve to explain the principles of the invention.

In the drawings:
FIG. 1A is a conceptual view illustrating an example of a health band terminal according to the present invention, viewed in one direction;
FIG. 1B is a perspective view illustrating an example of a health band terminal 100 according to another embodiment of the present invention;
FIG. 1C is a conceptual view illustrating one area of a health band terminal 100 provided with first and second electrode portions;
FIGS. 2A to 2C are views illustrating a structure of a body component collecting sensor unit according to one embodiment of the present invention;
FIGS. 3A to 3C are views illustrating a structure of a body component collecting sensor unit according to another embodiment of the present invention;
FIG. 3D is a conceptual view illustrating an arrangement structure of an electrode in accordance with still another embodiment of the present invention;
FIGS. 4A and 4B are conceptual views illustrating a structure of a second electrode portion arranged together with a push key;
FIGS. 5A and 5B are conceptual views illustrating types of first and second electrodes if there is no user input unit in a main body;
FIGS. 6A and 6B are conceptual views illustrating an arrangement structure of a user input unit and a second electrode portion;
FIGS. 7A and 7B are conceptual views illustrating an arrangement structure of a second electrode portion arranged to adjoin a user input unit;
FIGS. 8A to 8C are conceptual views illustrating an arrangement structure of a user input unit and a second electrode portion;
FIGS. 9A to 9C are conceptual views illustrating a key electrode module provided with a user input unit and a second electrode portion, which are formed in a single body;
FIG. 9D is a conceptual view illustrating a structure of a first electrode and an actuator in a key electrode module according to various embodiments;
FIGS. 10A and 10B are conceptual views illustrating a connection structure of a main circuit board and an electrode portion;
FIGS. 11A to 11E are conceptual views illustrating a structure that a main circuit board and an electrode portion are directly connected with each other in accordance with another embodiment;
FIGS. 12A to 12D are conceptual views illustrating a control method for outputting touch guide information of a finger through a display unit;
FIG. 13 is a flow chart illustrating a control method for controlling a body component measurement function on the basis of an area of a finger, which touches an electrode portion, in accordance with one embodiment;
FIGS. 14A and 14B are conceptual views illustrating a control method for controlling a health band terminal by using an electrode portion; and
FIG. 15 is a flow chart illustrating a control method for applying different body fat algorithms on the basis of a skin color.

### DETAILED DESCRIPTION OF THE PREFERRED EMBODIMENT

Description will now be given in detail according to exemplary embodiments disclosed herein, with reference to the accompanying drawings. For the sake of brief description with reference to the drawings, the same or equivalent components may be provided with the same or similar reference numbers, and description thereof will not be repeated. In general, a suffix such as "module" and "unit" may be used to refer to elements or components. Use of such a suffix herein is merely intended to facilitate description of the specification, and the suffix itself is not intended to give any special meaning or function. In the present disclosure, that which is well-known to one of ordinary skill in the relevant art has generally been omitted for the sake of brevity. The accompanying drawings are used to help easily understand various technical features and it should be understood that the embodiments presented herein are not limited by the accompanying drawings. As such, the present disclosure should be construed to extend to any alterations, equivalents and substitutes in addition to those which are particularly set out in the accompanying drawings.

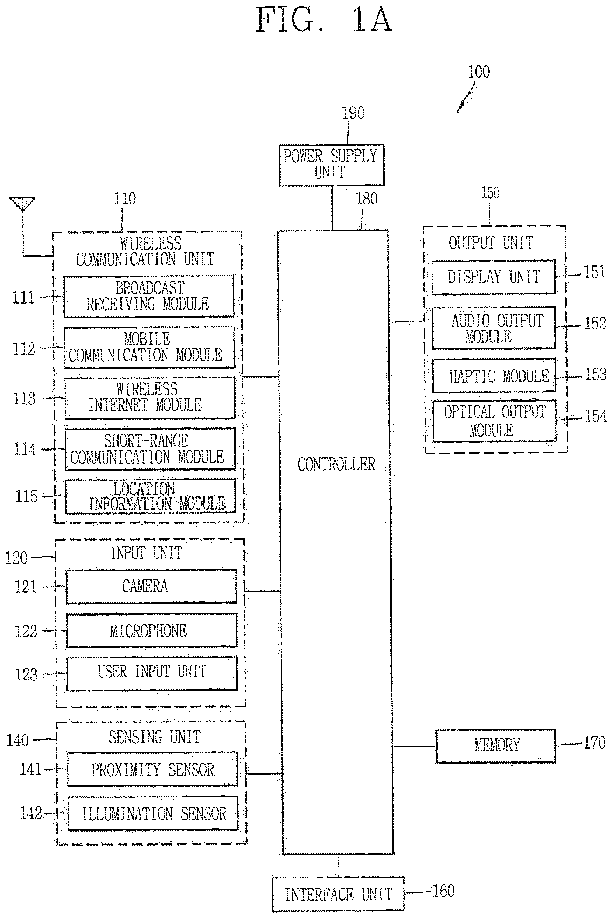

FIG. 1A is a conceptual view of an example of a health band terminal according to an embodiment of the present invention, which is viewed from one direction.

The health band terminal 100 is shown having components such as a wireless communication unit 110, an input unit 120, a sensing unit 140, an output unit 150, an interface unit 160, a memory 170, a controller 180, and a power supply unit 190. It is understood that implementing all of the illustrated components is not a requirement, and that greater or fewer components may alternatively be implemented.

The health band terminal 100 is shown having wireless communication unit 110 configured with several commonly implemented components. For instance, the wireless communication unit 110 typically includes one or more components which permit wireless communication between the health band terminal 100 and a wireless communication system or network within which the health band terminal is located.

The wireless communication unit 110 typically includes one or more modules which permit communications such as wireless communications between the health band terminal 100 and a wireless communication system, communications between the health band terminal 100 and another health band terminal, communications between the health band terminal 100 and an external server. Further, the wireless communication unit 110 typically includes one or more modules which connect the health band terminal 100 to one or more networks. To facilitate such communications, the wireless communication unit 110 includes one or more of a broadcast receiving module 111, a mobile communication module 112, a wireless Internet module 113, a short-range communication module 114, and a location information module 115.

The input unit 120 includes a camera 121 for obtaining images or video, a microphone 122, which is one type of audio input device for inputting an audio signal, and a user input unit 123 (for example, a touch key, a push key, a mechanical key, a soft key, and the like) for allowing a user to input information. Data (for example, audio, video, image, and the like) is obtained by the input unit 120 and may be analyzed and processed by controller 180 according to device parameters, user commands, and combinations thereof.

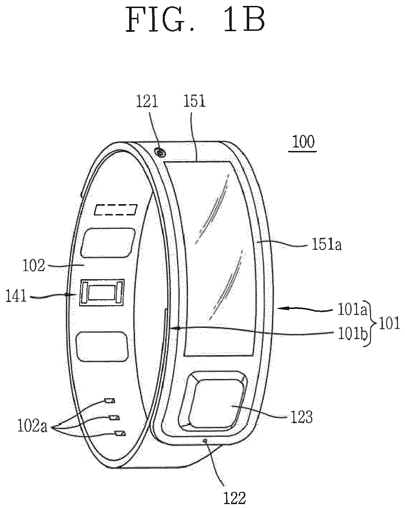

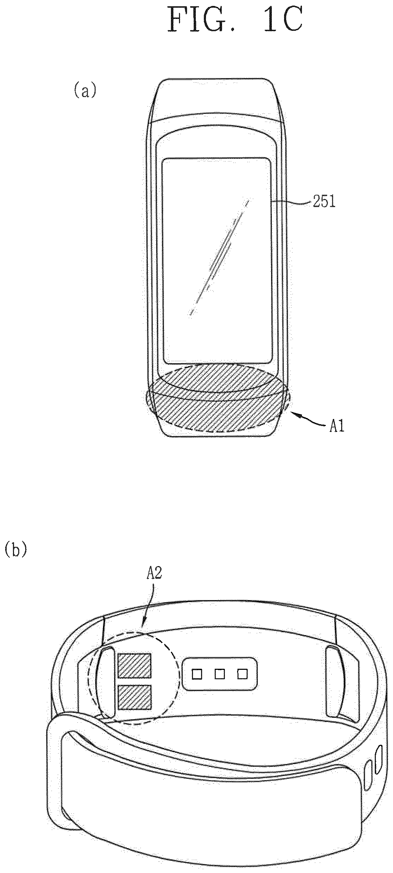

The sensing unit 140 is typically implemented using one or more sensors configured to sense internal information of the health band terminal, the surrounding environment of the health band terminal, user information, and the like. For example, in FIG. 1, the sensing unit 140 is shown having a proximity sensor 141 and an illumination sensor 142. If desired, the sensing unit 140 may alternatively or additionally include other types of sensors or devices, such as a touch sensor, an acceleration sensor, a magnetic sensor, a G-sensor, a gyroscope sensor, a motion sensor, an RGB sensor, an infrared (IR) sensor, a finger scan sensor, a ultrasonic sensor, an optical sensor (for example, camera 121), a microphone 122, a battery gauge, an environment sensor (for example, a barometer, a hygrometer, a thermometer, a radiation detection sensor, a thermal sensor, and a gas sensor, among others), and a chemical sensor (for example, an electronic nose, a health care sensor, a biometric sensor, and the like), to name a few. The health band terminal 100 may be configured to utilize information obtained from sensing unit 140, and in particular, information obtained from one or more sensors of the sensing unit 140, and combinations thereof.

The output unit 150 is typically configured to output various types of information, such as audio, video, tactile output, and the like. The output unit 150 is shown having a display unit 151, an audio output module 152, a haptic module 153, and an optical output module 154.

The display unit 151 may have an inter-layered structure or an integrated structure with a touch sensor in order to facilitate a touch screen. The touch screen may provide an output interface between the health band terminal 100 and a user, as well as function as the user input unit 123 which provides an input interface between the health band terminal 100 and the user.

The interface unit 160 serves as an interface with various types of external devices that can be coupled to the health band terminal 100. The interface unit 160, for example, may include any of wired or wireless ports, external power supply ports, wired or wireless data ports, memory card ports, ports for connecting a device having an identification module, audio input/output (I/O) ports, video I/O ports, earphone ports, and the like. In some cases, the health band terminal 100 may perform assorted control functions associated with a connected external device, in response to the external device being connected to the interface unit 160.

The memory 170 is typically implemented to store data to support various functions or features of the health band terminal 100. For instance, the memory 170 may be configured to store application programs executed in the health band terminal 100, data or instructions for operations of the health band terminal 100, and the like. Some of these application programs may be downloaded from an external server via wireless communication. Other application programs may be installed within the health band terminal 100 at time of manufacturing or shipping, which is typically the case for basic functions of the health band terminal 100 (for example, receiving a call, placing a call, receiving a message, sending a message, and the like). It is common for application programs to be stored in the memory 170, installed in the health band terminal 100, and executed by the controller 180 to perform an operation (or function) for the health band terminal 100.

The controller 180 typically functions to control overall operation of the health band terminal 100, in addition to the operations associated with the application programs. The controller 180 may provide or process information or functions appropriate for a user by processing signals, data, information and the like, which are input or output by the various components depicted in FIG. 1A, or activating application programs stored in the memory 170. As one example, the controller 180 controls some or all of the components according to the execution of an application program that have been stored in the memory 170.

The power supply unit 190 can be configured to receive external power or provide internal power in order to supply appropriate power required for operating elements and components included in the health band terminal 100. The power supply unit 190 may include a battery, and the battery may be configured to be embedded in the terminal body, or configured to be detachable from the terminal body.

At least some of the components may operate in cooperation with one another in order to implement an operation, control or control method of the health band terminal according to various exemplary embodiments to be described below. The operation, control or control method of the health band terminal may be implemented on the health band terminal by the execution of at least one application program stored in the memory 170.

FIG. 1B is a conceptual view of an example of a health band terminal according to anther embodiment of the present invention.

Referring to FIG. 1B, the health band terminal 100 includes a main body 101 with a display unit 151 and a band 102 connected to the main body 101 to be wearable on a wrist. The main body 101 may include a case having a certain appearance. As illustrated, the case may include a first case 101a and a second case 101b cooperatively defining an inner space for accommodating various electronic components. Other configurations are possible. For instance, a single case may alternatively be implemented, with such a case being configured to define the inner space, thereby implementing a health band terminal 100 with a uni-body.

The health band terminal 100 can perform wireless communication, and an antenna for the wireless communication can be installed in the main body 101. The antenna may extend its function using the case. For example, a case including a conductive material may be electrically connected to the antenna to extend a ground area or a radiation area.

The display unit 151 is shown located at the front side of the main body 101 so that displayed information is viewable to a user. In some embodiments, the display unit 151 includes a touch sensor so that the display unit can function as a touch screen. As illustrated, window 151a is positioned on the first case 101a to form a front surface of the terminal body together with the first case 101a.

The illustrated embodiment includes audio output module 152, a camera 121, a microphone 122, and a user input unit 123 positioned on the main body 101. When the display unit 151 is implemented as a touch screen, additional function keys may be minimized or eliminated. For example, when the touch screen is implemented, the user input unit 123 may be omitted.

The band 102 is commonly worn on the user's wrist and may be made of a flexible material for facilitating wearing of the device. As one example, the band 102 may be made of fur, rubber, silicon, synthetic resin, or the like. The band 102 may also be configured to be detachable from the main body 101. Accordingly, the band 102 may be replaceable with various types of bands according to a user's preference.

In one configuration, the band 102 may be used for extending the performance of the antenna. For example, the band may include therein a ground extending portion (not shown) electrically connected to the antenna to extend a ground area.

The band 102 may include fastener 102a. The fastener 102a may be implemented into a buckle type, a snap-fit hook structure, a Velcro® type, or the like, and include a flexible section or material. The drawing illustrates an example that the fastener 102a is implemented using a buckle.

A health band terminal 100 according to one embodiment of the present invention comprises a body component collecting sensor 200. The body component collecting sensor 200 according to the present invention measures an impedance value for one area of a body by using a plurality of electrodes touched with different areas of the body. Body fat data of the body may be acquired based on the impedance value and other information of the body. To measure the impedance value, the body component collecting sensor 200 of the health band terminal 100 includes first and second electrode portions which are simultaneously in contact with different areas of the user body.

FIG. 1C is a conceptual view illustrating one area of the health band terminal 100 provided with the first and second electrode portions. Referring to (a) of FIG. 1C, the first electrode portion may be arranged on a first area A1. The first area A1 corresponds to an area which does not face a wrist of a user if the health band terminal 100 is worn on the wrist of the user. For example, the first area A1 may be arranged at an area adjacent to the display unit 151.

Meanwhile, the second electrode portion is arranged on a second area A2. The second area A2 corresponds to an area which is in contact with the wrist of the user in a state that the health band terminal 100 is worn on the wrist of the user. For example, the second electrode portion may be formed on one area of the other surface facing one surface where the display unit is formed, or may be formed on a band 102 configured to surround the wrist.

The second electrode portion formed on the second area A2 is in contact with the body when the user wears the health band terminal 100, and the first electrode portion formed on the first area A1 should intentionally be in contact with a finger, etc. of the user in a state that health band terminal 100 is worn on the wrist of the user. If the body of the user touches the first and second electrode portions at the same time, the controller 180 may form body component data by using the measured impedance value.

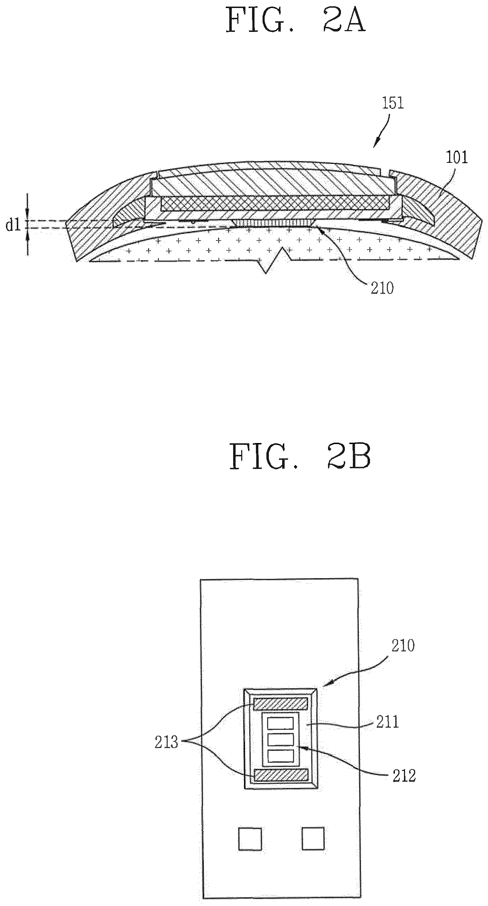

FIGS. 2A to 2C are views illustrating a structure of a body component collecting sensor unit according to one embodiment of the present invention.

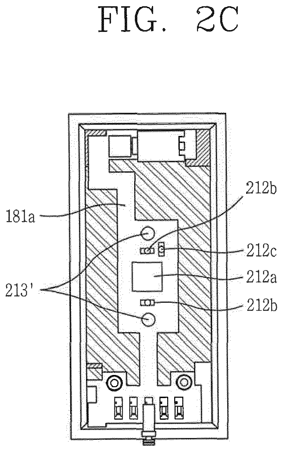

FIG. 2A is a cross-sectional view of one area of the health band terminal 100, FIG. 2b is a conceptual view illustrating an arrangement structure of a PPG sensor unit and a body component measurement sensor module, and FIG. 2C is a view illustrating an arrangement structure of components of a body component collecting sensor.

The body component collecting sensor unit 210 according to FIGS. 2A to 2C is arranged on one surface of the main body 101. The body component collecting sensor unit 210 is arranged to face the display unit 151, and is arranged on an area with which the wrist is in contact if the health band terminal 100 is worn on the wrist of the user.

The body component collecting sensor unit 210 is protruded from the outer surface of the main body 101, and includes a protrusion 211, a PPG sensor unit 212 arranged on the protrusion 211, and a pair of electrodes 213.

The protrusion 211 is protruded from the outer surface of the main body 101 as much as a predetermined thickness d1. Therefore, the surface of the protrusion 211 may be tightly adhered to the wrist of the user. The PPG sensor unit 212 is formed on one area of the protrusion 211. The PPG sensor unit 212 may include a light receiving sensor 212a, a pair of green light emitting diodes 212b and an IR sensor 211c.

Referring to FIG. 2C, the pair of green light emitting diodes 212b are arranged to be spaced apart from each other based on the light receiving sensor 211a, and the IR sensor 212c is arranged to adjoin one of the green light emitting diodes 212b. Light emitted from the IR sensor 212c and the green light emitting diodes 212b is reflected by the body of the user and received in the light receiving sensor 212a. The light receiving sensor 212c may determine whether the user wears the health band terminal 100 or acquire heartbeat information of the user, on the basis of the amount of the received light.

Since components of the PPG sensor unit 212 are formed on the protrusion 211, the components may be tightly adhered to the wrist of the user. Therefore, leakage of the light may be minimized, whereby accuracy of measurement may be improved.

Meanwhile, a pair of connection terminals 213' for measuring the impedance value for body component measurement are arranged to be spaced apart from each other by interposing the PPG sensor unit 212 therebetween. That is, one connection terminal 213' is arranged to adjoin one green light emitting diode 212b, and the other connection terminal 213' is arranged to adjoin the other green light emitting diode 212b. The pair of connection terminals 213' correspond to the pair of electrodes 213, and are electrically connected with the pair of electrodes 213.

Referring to FIG. 2B again, the pair of electrodes 213 are formed on one surface of the protrusion 211, which is protruded. Therefore, if the health band terminal 100 is worn on the wrist of the user, the pair of electrodes 213 may be tightly adhered to the wrist of the user. Each of the pair of connection terminals 213' is comprised of pogo pin, and may electrically connect the pair of electrodes 213 formed on the protrusion 211 protruded from the outer surface of the main body 101 with a circuit board 180a arranged inside the main body 101.

According to this embodiment, the PPG sensor unit 212 for collecting body data and the pair of electrodes 213 for collecting body component information are all formed on the protrusion formed to easily touch the body of the user when the user wears the health band terminal 100. Therefore, measurement accuracy of the PPG sensor unit 212 is not deteriorated and at the same time the skin of the user may be tightly adhered to the pair of electrodes 213, whereby the impedance value may be measured exactly.

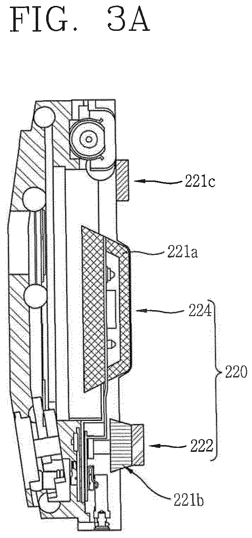

FIGS. 3A to 3C are views illustrating a structure of a body component collecting sensor unit according to another embodiment of the present invention.

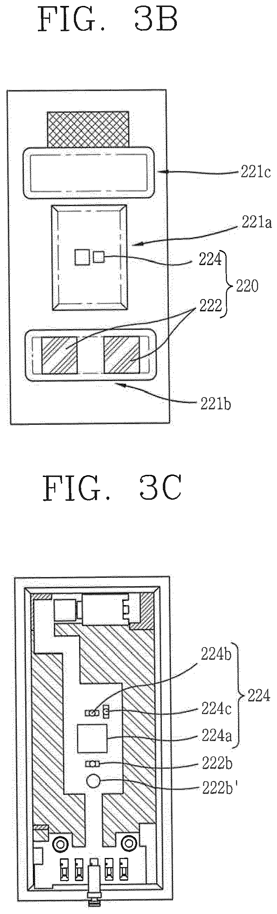

FIG. 3A is a cross-sectional view illustrating one area of the health band terminal 100, FIG. 3B is a conceptual view illustrating an arrangement structure of PPG sensor unit and a body component measurement sensor module, and FIG. 3C is a view illustrating an arrangement structure of components of a body component collecting sensor.

The body component collecting sensor unit 220 according to FIGS. 3A to 3C includes a PPG sensor unit 224 formed on a first protrusion 221a and an electrode 222 formed on a second protrusion 221b. The first protrusion 221a is formed in a state that it is protruded from one surface of the main body 101, whereby the first protrusion 221a is tightly adhered to the wrist of the user.

The PPG sensor unit 224 includes a light receiving sensor 224a, a pair of green light emitting diodes 224b, and an IR sensor 224c, and components of the PPG sensor unit 224 are formed on the first protrusion 221.

If the first protrusion 221a is arranged at the center of one surface of the main body 101, the second protrusion 221b is arranged to adjoin the first protrusion 221a. The pair of electrodes 222 are formed on the second protrusion 221b. The pair of electrodes 222 are arranged to be spaced apart from each other. It is preferable that the first protrusion 221a and the second protrusion 221b are formed to have substantially the same thickness as each other or are formed to have a narrow thickness.

Meanwhile, the health band terminal 100 according to this embodiment further includes a dummy protrusion 221c arranged to be spaced apart from the second protrusion 221b by interposing the first protrusion 221a therebetween. That is, the second protrusion 221b, the first protrusion 221a and the dummy protrusion 221c are arranged in a line. The dummy protrusion 221c may have substantially the same thickness as that of the second protrusion 221b. Therefore, in a state that the health band terminal 100 is worn on the wrist of the user, the first and second protrusions 221a and 221b and the dummy protrusion 221c may simultaneously be in contact with the body of the user.

According to this embodiment, in a state that the first and second protrusions 221a and 221b are in contact with the body of the user, the other area of the main body is stably supported by the dummy protrusion 221c, whereby stable wearing may be provided.

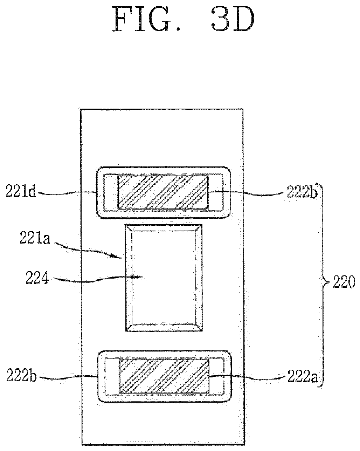

FIG. 3D is a conceptual view illustrating an arrangement structure of electrodes in accordance with still another embodiment.

Referring to FIG. 3D, the health band terminal 100 according to this embodiment includes first to third protrusions 221a, 221b and 221c arranged on one surface of the main body 101 along one direction. The third and third protrusions 221b and 221c are arranged to be spaced apart from each other by interposing the first protrusion 221a therebetween. The PPG sensor unit 224 is arranged on the first protrusion 221a.

The first and second electrodes 222a and 222b for acquiring an impedance value of one area of the body of the user are formed on the second and third protrusions 221b and 221c, respectively. It is preferable that the second and third protrusions 221b and 221c are formed to have substantially the same thickness.

Since first and second electrodes 222a and 222b are formed on their respective protrusions different from each other, their widths may be enlarged within a maximum range. Therefore, since a current may flow to a wider body by means of the wider electrodes, the impedance value may be acquired more stably.

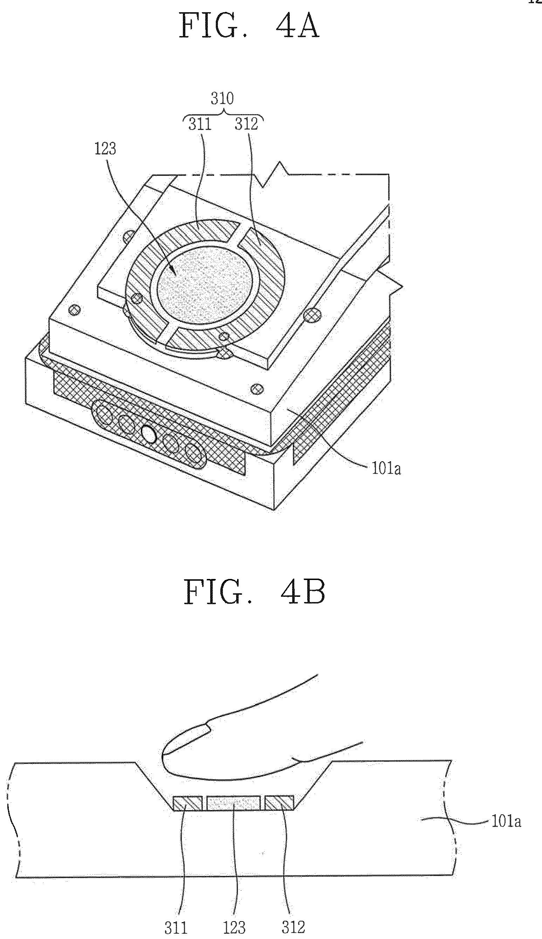

FIGS. 4A and 4B are conceptual views illustrating a structure of a second electrode portion arranged together with a push key.

Referring to FIGS. 1B and 4A, the first electrode portion is formed on one surface of the main body 101 facing the body of the user if the health band terminal 100 is worn on the body of the user as shown in FIGS. 2A to 3D. Meanwhile, the second electrode portion is formed on one surface exposed to the outside if the health band terminal 100 is worn on the body of the user.

For example, first and second electrodes 311 and 312 of a second electrode portion 310 are formed to adjoin the user input unit 123 comprised of a push key. The first and second electrodes 311 and 312 may respectively serve as a current electrode for applying a current to a body of a user and a voltage electrode for measuring impedance.

The user input unit 123 may be implemented to form a control signal in accordance with a push caused by an external force. The first and second electrodes 311 and 312 may be formed along the rim of the user input unit 123. For example, if the user input unit 123 includes a circular key, the first and second electrodes 311 and 312 may be formed along an outer circumference of the circular key. The first and second electrodes 311 and 312 are formed so as not to adjoin each other. The circular key and a first case 101a of the main body 10, which is arranged between the first and second electrodes 311 and 312, are made of insulating members.

Preferably, an area of the first and second electrodes 311 and 312 surrounding the user input unit 123 is not greater than, but not limited to, a width of a finger of a user. Also, the first and second electrodes 311 and 312 and the user input unit 123 may be formed to constitute one surface.

According to the present invention, the first and second electrodes 311 and 312 of the first electrode portion 310 are arranged to adjoin each other. Therefore, since one finger may touch both the first and second electrodes 311 and 312, it is not required for the body of the user to touch the two electrodes by using two or more fingers. Therefore, since the body of the user may touch the first and second electrodes while maintaining a pose more stably, a proper pose may be maintained while body component information is being acquired.

FIG. 4B is a cross-sectional view illustrating that the first case 101a on which the user input unit and the first and second electrodes 311 and 312 are arranged is cut. Referring to FIG. 4B, one area of the first case 101a where the first and second electrodes 311 and 312 and the user input unit 123 are formed may be formed to be more recessed than the other areas.

Since the first and second electrodes 311 and 312 and the user input unit 123 are more recessed than the other areas of the first case 101a, a finger of the user may touch the first and second electrodes 311 and 312 more exactly by the sense of touch. Therefore, the problem that the finger of the user touches one area only of the first and second electrodes 311 and 312 may be avoided.

The first and second electrodes 311 and 312 and the user input unit 123 are arranged on the same surface as the recessed area of the first case 101a on the drawing but their arrangement is not limited to this case. For example, the first and second electrodes 311 and 312 may be arranged on a side (inclined surface) that forms the recessed area of the first case 101a.

The user may allow the finger to touch a full area of the first and second electrodes 311 and 312 by tactually perceiving the recessed area of the main body 101 and arranging the finger on the recessed area. Therefore, the impedance value may be acquired more exactly.

The second electrode portion according to the present invention is formed on one area of the main body exposed to the outside if the health band terminal is worn on the body of the user. Preferably, the second electrode portion is formed on one surface where it is easy for the user to touch with the finger (one surface where the display unit 151 is arranged). Hereinafter, a type and arrangement structure of a pair of electrodes of the first electrode portion will be described.

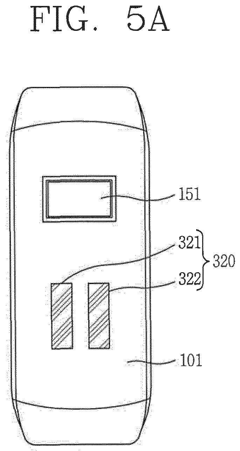

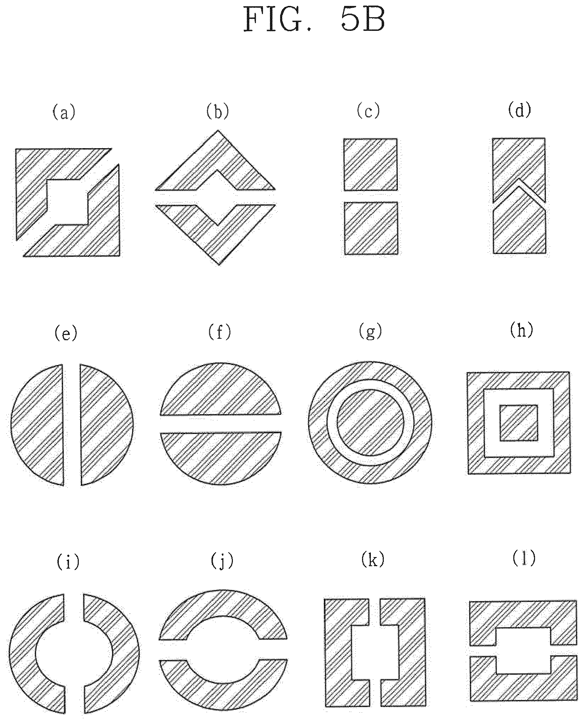

FIGS. 5A and 5B are conceptual views illustrating types of first and second electrodes if there is no user input unit in a main body.

Referring to FIG. 5A, the display unit 151 and the second electrode portion 320 are arranged on one surface of the terminal main body 101. The second electrode portion 320 may be comprised of first and second electrodes 321 and 322 formed in parallel in a bar shape. The first and second electrodes 321 and 322 are formed in the same shape as each other.

The first and second electrodes 321 and 322 are formed of an area within a specific range, and have a spaced interval so as not to adjoin each other. For example, the area may correspond to 30mm' to 80mm', and the spaced interval is preferably formed between 1mm and 2mm.

Referring to FIG. 5B, the first and second electrodes may have various shapes. The first and second electrodes are spaced apart from each other and do not adjoin each other. Also, the first and second electrodes substantially have the same shape or have shapes corresponding to each other.

Referring to (a) of FIG. 5B, the first and second electrodes have a square shape on the whole, and further include a square shaped spaced area at a center area. Referring to (b) of FIG. 5B, the first and second electrodes have a diamond shape, and further include a diamond shaped spaced area at a center area.

Referring to (c) of FIG. 5B, the first and second electrodes respectively have square shapes, and are arranged in a line while forming a spaced area from each other. Referring to (d) of FIG. 5B, the first and second electrodes may be formed to correspond to each other such that the spaced area may have a specific shape.

Referring to (e) and (f) of FIG. 5B, the first and second electrodes have a semicircle shape, and a spaced area between the first and second electrodes may be formed in a bar shape.

Referring to (g) and (h) of FIG. 5B, the first electrode may have a circle shape or a square shape, and the second electrode may be formed in a closed loop structure formed along the edge of the first electrode.

Referring to (i) and (j) of FIG. 5B, the first and second electrodes may be formed in a curved structure that they are curved to face each other, such that a spaced area between the first and second electrodes may have a circle shape.

Referring to (k) and (l) of FIG. 5B, the first and second electrodes may be formed in a bent structure that they are symmetrical to each other, such that a spaced area between the first and second electrodes may have a square shape.

That is, the first and second electrodes are arranged to adjoin each other and insulated from each other to have various structures. Although not shown, other electronic components may be arranged at the spaced area of the first and second electrodes.

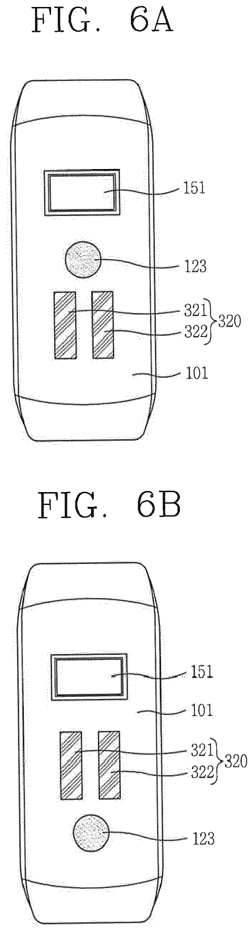

FIGS. 6A and 6B are conceptual views illustrating an arrangement structure of a user input unit and a second electrode portion.

Referring to FIGS. 6A and 6B, the display unit 151, the user input unit 123 and the second electrode portion 320 may be arranged along one direction. The arrangement order of the user input unit 123 and the second electrode portion 320 may be changed.

Even in this case, the second electrode portion 320 may have various shapes shown in FIG. 5B.

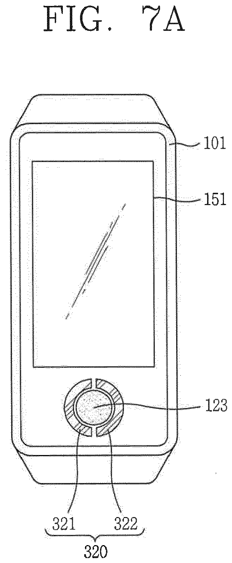

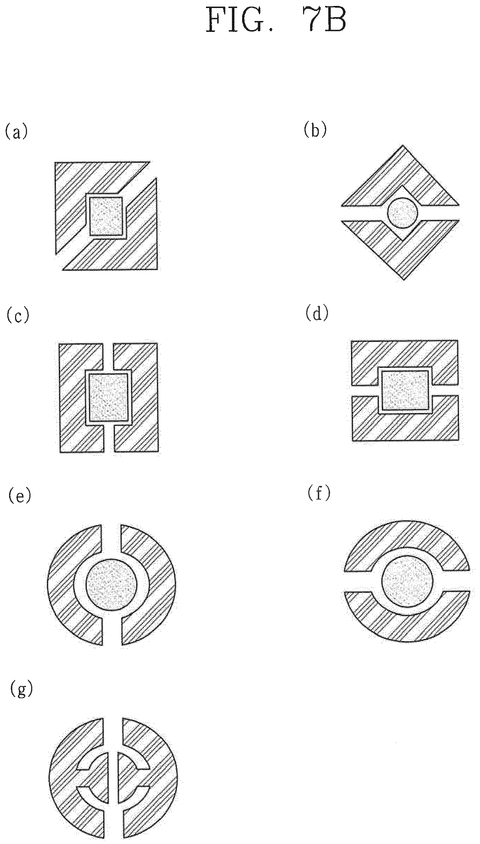

FIGS. 7A and 7B are conceptual views illustrating an arrangement structure of a second electrode portion arranged to adjoin a user input unit.

Referring to FIG. 7A, the user input unit 123 and the second electrode portion 320 are arranged on one surface where the display unit 151 of the main body 101 is arranged. The second electrode portion 320 includes first and second electrodes 321 and 322 that form the spaced area. The user input unit 123 is formed between the first and second electrodes 321 and 322.

FIG. 7B illustrates various structures that the user input unit is arranged between the first and second electrodes.

Referring to (a) and (b) of FIG. 7B, a square shaped spaced area is formed between the first and second electrodes. The user input unit 123 is arranged on the spaced area. The user input unit 123 may be formed of, but not limited to, a key button of which section has a circle shape.

Referring to (c) and (d) of FIG. 7B, the first and second electrodes are formed in a '⊏' shape to face each other, and form a square shaped spaced area. The user input unit 123 may be formed of a square shaped key button corresponding to the spaced area.

Referring to (e) and (f) of FIG. 7B, the first and second electrodes are formed in a 'c' shape to face each other, thereby forming a circle shaped spaced area. A key button of the user input unit 123 arranged at the spaced area may be formed in a circle shape.

Referring to (g) of FIG. 7B, the 'E' shaped first and second electrodes are arranged to face each other, and although not shown in detail, a push key formed to apply a control command by means of a push may be arranged at a center area.

According to these embodiments, since the user input unit is formed at the space between the first and second electrode portions, efficiency in space utility may be improved.

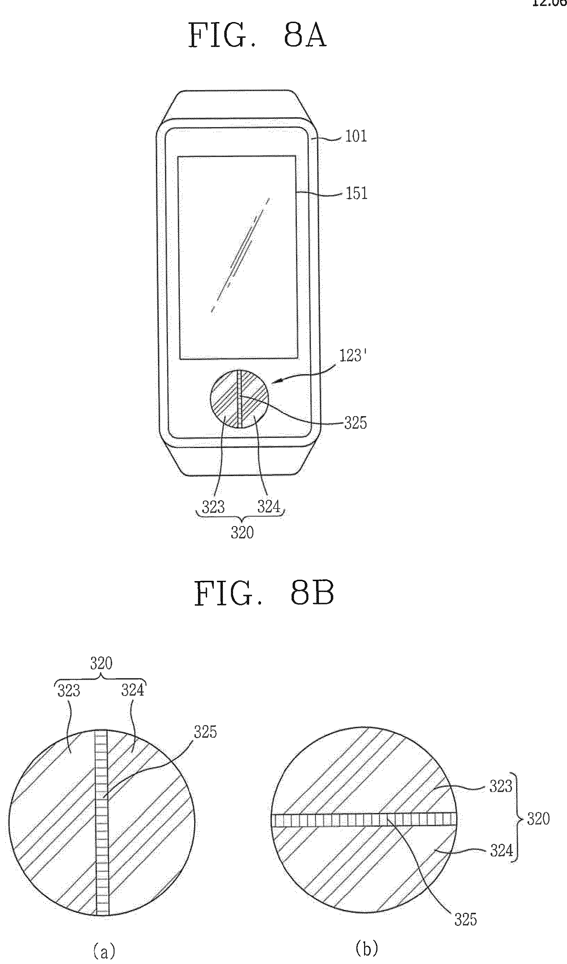

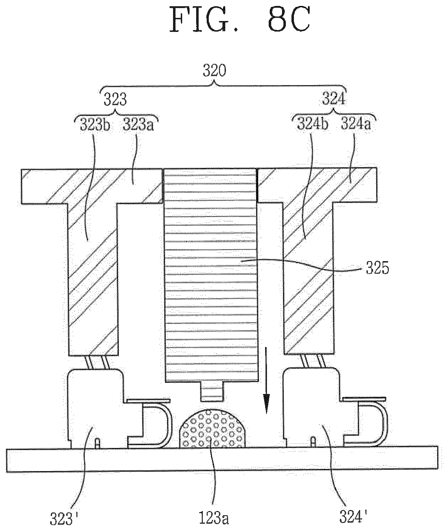

FIGS. 8A to 8C are conceptual views illustrating an arrangement structure of a user input unit and a second electrode portion.

The user input unit and the second electrode portion according to this embodiment are formed in a single body, and will be referred to as a key electrode module 123'. The key electrode module 123' includes first and second electrodes 323 and 324. The first electrode 323 includes a first area 323a exposed to the outside of the main body 101 and a second area 323b extended from the first area 323a toward the inside of the main body 101. The second electrode 324 includes a first area 324a exposed to the outside of the main body 101 and a second area 324b extended from the first area 324a toward the inside of the main body 101.

A push key 325 is formed between the first area 323a of the first electrode 323 and the first area 324a of the second electrode 324. The push key 325 is arranged between the first and second electrodes 323 and 324, and is made of a nonconductor to insulate the first and second electrodes 323 and 324 from each other. The first areas 323a and 324a of the first and second electrodes 323 and 324 are coupled to an end of the push key 325. The end of the push key 325 is exposed to the outside of the main body 101 together with the first areas 323a and 324a, and the push key 325 is extended toward the inside of the main body 101.

First and second connectors 323' and 324' are arranged on a circuit board 180a arranged inside the main body 101, and an actuator 123a is formed between the first and second connectors 323' and 324'. The first and second connectors 323' and 324' are electrically connected with ends of the second areas 323b and 324b of the first and second electrodes 323 and 324. Meanwhile, the other end of the push key 325 is arranged to face the actuator 123a. If the actuator 123a is pushed by the push key 325, a control signal is formed.

If the finger of the user touches the first and second electrodes 323 and 324, a current flows to the finger of the user, and the controller 180 may calculate an impedance value. Meanwhile, if an external force is applied to the first and second electrodes 323 and 324 and/or the push key 325, the first and second electrodes 323 and 324 and the push key 325 move together. Therefore, the actuator 123a is pushed by the push key 325 and thus the control signal is generated.

Referring to (a) of FIG. 8B, the first and second electrodes 323 and 324 and the push key 325, which constitute external appearance of the main body, may have a circle shape. The end of the push key 325 may be formed in, but not limited to, a bar shape extended in one direction.

Also, referring to (b) of FIG. 8B, the push key 325 may be implemented in various directions.

If the finger of the user touches the key electrode module according to this embodiment, the impedance value may be acquired by the first and second electrodes 323 and 324. Meanwhile, if the push key 325 moves as an external force is applied to the key electrode module, the control command may be input. In this case, the impedance value may be measured to acquire body component data while the control command is being applied.

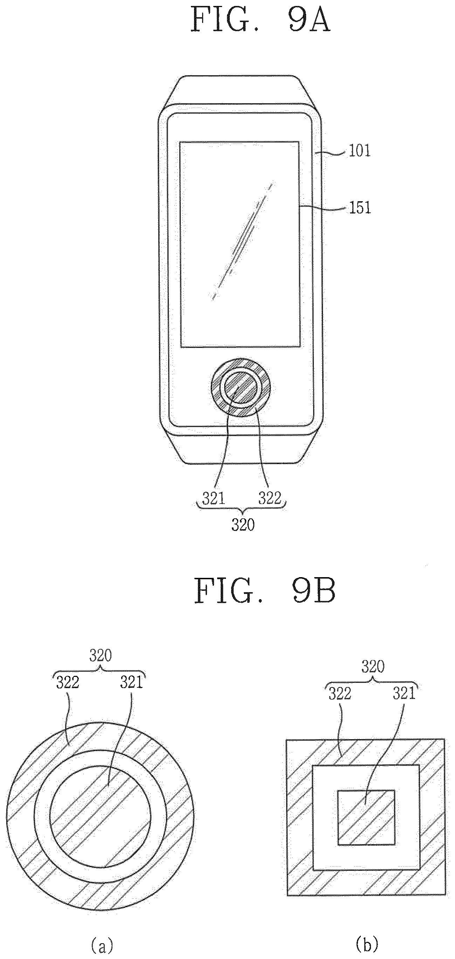

FIGS. 9A to 9C are conceptual views illustrating a key electrode module provided with a user input unit and a second electrode portion, which are formed in a single body.

Referring to FIGS. 9A and 9B, the second electrode portion includes a first electrode 321 and a second electrode 322 formed to surround the first electrode 321. Referring to (a) of FIG. 9B, if a section of the first electrode 321 has a circle shape, a section of the second electrode 322 may also have a circular stripe shape. Referring to (b) of FIG. 9B, if a section of the first electrode 321 has a square shape, a section of the second electrode 322 may also have a square stripe shape.

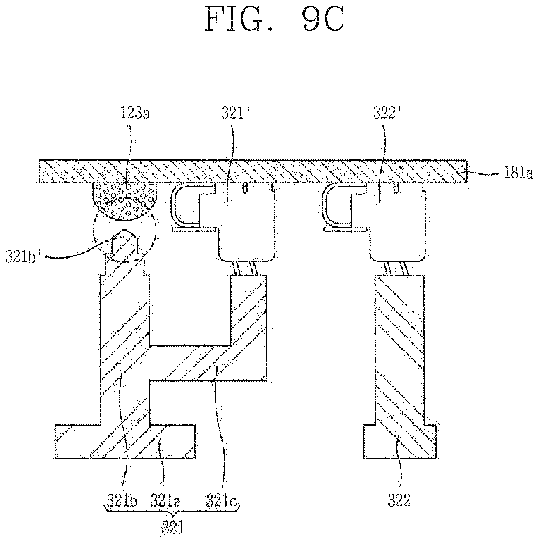

FIG. 9C is a conceptual view illustrating a structure of a key electrode module 123 according to one embodiment of the present invention.

Referring to FIG. 9C, first and second connectors 321' and 322' and an actuator 123a are arranged on the circuit board 180a.

The first electrode 321 includes a first area 321a exposed to the outside of the main body 101, a second area 321b extended from the first area 321a toward the inside of the main body 101, and a third area 321c bent and extended from the second area 321b. An end 321b' of the second area 321b is arranged to face the actuator 123a. The end 321b' includes an extension portion of which section becomes narrow to facilitate modification of the actuator 123a by means of movement. An end of the third area 321c is electrically connected with the first connector 321'.

Meanwhile, one end of the second electrode 322 is exposed to the outside of the main body 101 and extended toward the inside of the main body 101. The other end of the second electrode 322 is electrically connected with the second connector 322'.

Therefore, an external force may be applied to the first electrode 321 (and the second electrode 322), whereby the control command may be applied. Also, if the external force is not applied, the finger of the user may touch the first and second electrodes 321 and 322 to acquire the impedance value.

According to this embodiment, it is not required that the structure of the push key for applying the control command should be formed separately from the components of the electrode portion. Therefore, the electrode portion and the user input unit may be formed at a narrow area of the main body.

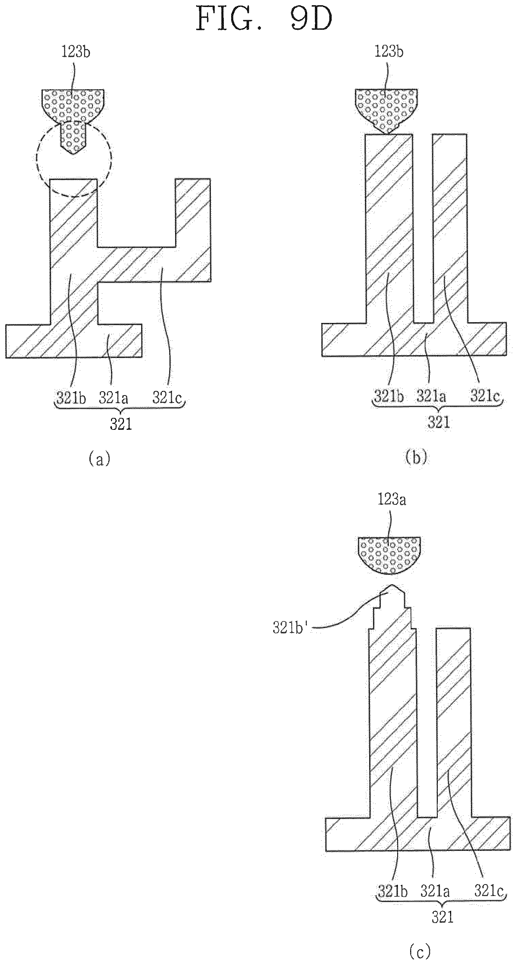

FIG. 9D is a conceptual view illustrating a structure of a first electrode and an actuator in a key electrode module according to various embodiments.

Referring to (a) of FIG. 9D, the first electrode 321 includes the first to third areas 321a, 321b and 321c. Since the first to third areas 321a, 321b and 321c are substantially the same as one another except the end 321b' of the third area 321b in the first to third areas 321a, 321b and 321c of FIG. 9c, their repeated description will be omitted.

The actuator 123b according to this embodiment includes an area that adjoins the second area 321b, wherein this area has a sharply protruded shape. Therefore, even though the end 321a' of the second area 321b has a stubby shape, the actuator 123b may apply the control command.

Referring to (b) of FIG. 9D, the second and third areas 321a and 321b are extended from the first area 321a in one direction. The second and third areas 321a and 321b may be formed in parallel. The end of the second area 321b is arranged to adjoin the actuator 123a, and the end of the third area 321c is electrically connected with the first connector 321'.

Referring to (c) of FIG. 9D, the second and third areas 321a and 321b are extended from the first area 321a in one direction. The end of the second area 321b is arranged to adjoin the actuator 123a, and the end of the third area 321c is electrically connected with the first connector 321'. However, the end of the second area 321b has a sharp shape of which section becomes narrow gradually. Therefore, a pressure may be applied to the actuator 123a more easily.

According to these embodiments, the user input unit and the body component measurement module may be implemented without a separate key module through the arrangement structure of the electrode portion and the actuator.

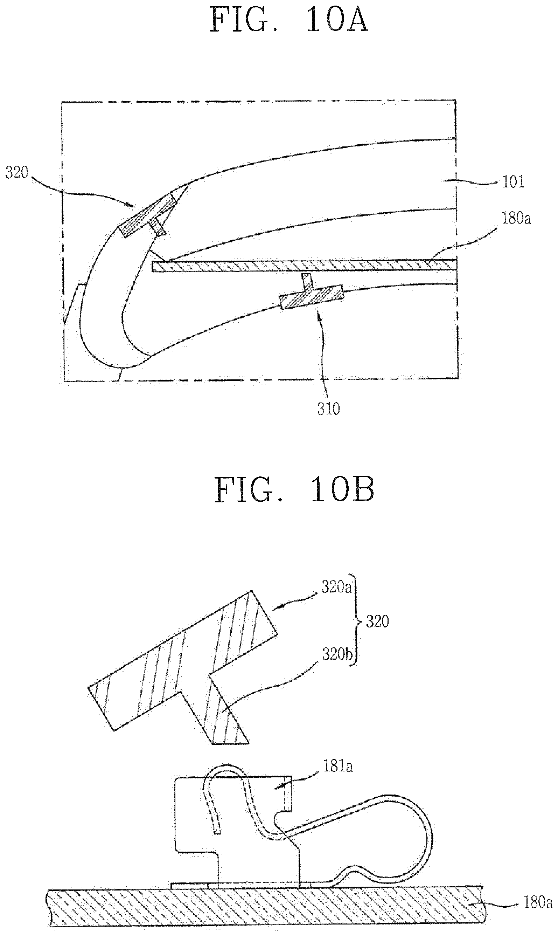

FIGS. 10A and 10B are conceptual views illustrating a connection structure of a main circuit board and an electrode portion.

Referring to FIG. 10A, the main circuit board 180a is arranged inside the main body 101. The first and second electrode portions 310 and 320 are arranged on their respective surfaces different from each other. The first and second electrode portions 310 and 320 are directly connected to the main circuit board 180a arranged inside the main body 101. The main circuit board 180a is provided with a bio processor (BP) chip for measuring the impedance value and forming body component data.

FIG. 10B is a conceptual view illustrating an example that the second electrode portion 320 is connected to the main circuit board 180a. The second electrode portion 320 includes a first area 320a exposed to the outside of the main body 101 and a second area 320b extended from the first area 320a and connected to the main circuit board 180a. The end of the second area 320b is connected to a c-clip type connector 181a.

If the first area 320a is installed in the main body 101 obliquely based on the main circuit board 180a, the second area 320b is extended vertically to the first area 320a. However, the end of the second area 320b may be formed in an obliquely cut shape to be connected with the connector 181a installed in the main circuit board 180a. Therefore, the end of the second area 320b may stably be in contact with the connector 181a of the c-clip type.

According to this embodiment, the electrodes constituting the first and second electrode portions may directly be in contact with the main circuit board for controlling the health band terminal 100. Therefore, since the electrodes may directly be connected to the bio processor without a separate connection line, resistance matching for matching impedance is not required.

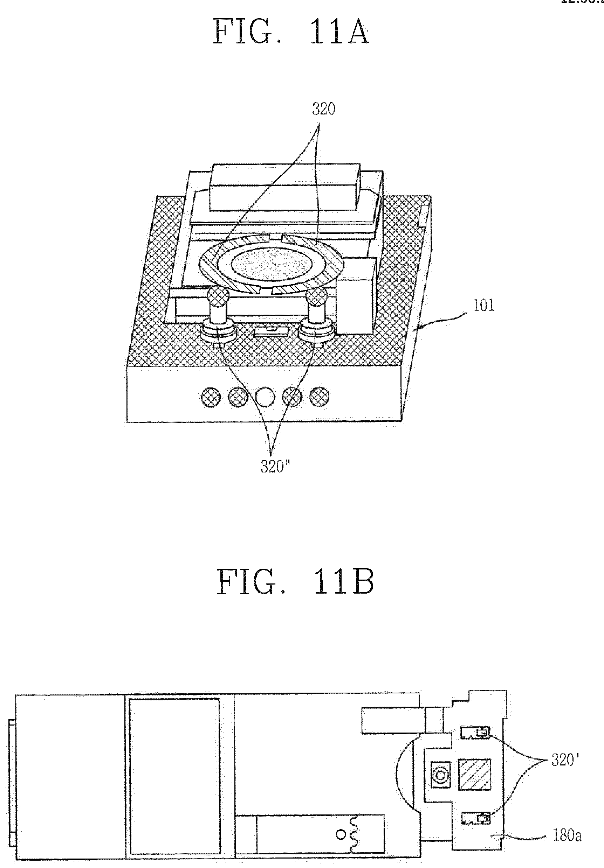

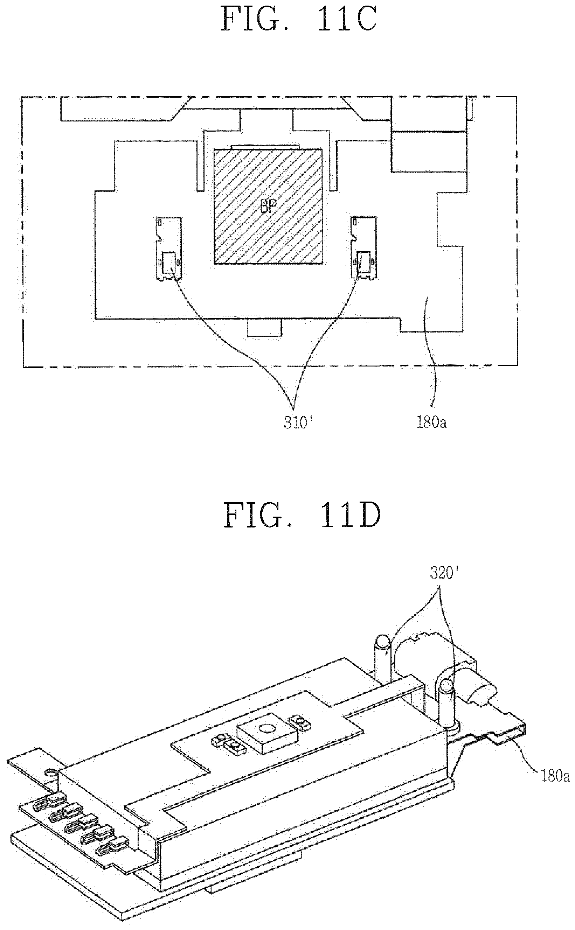

FIGS. 11A to 11E are conceptual views illustrating a structure that a main circuit board and an electrode portion are directly connected with each other in accordance with another embodiment.

Since the first and second electrode portions 310 and 320 are arranged on the main circuit board 180a together with the bio processor (BP) chip, the first and second electrode portions 310 and 320 may directly be connected with the bio processor (BP) chip without a separate connection line.

FIGS. 11A to 11C are conceptual views illustrating an arrangement structure of the first electrode portion arranged on a front surface of the main body. A first connector 310' for touching the first and second electrodes of the first electrode portion 310 is formed on one surface of the main circuit board 180a. The first connector 310' is formed to correspond to the number of the electrodes of the first electrode portion 310.

An extension portion 320" for connecting the first and second electrodes with the first connector 310' is formed to be exposed to the surface of the main body 101. The extension portion 320" may be made of the same material as that of the first and second electrodes, or may be implemented as additional connection terminal.

The bio processor (BP) chip is formed on one surface of the main circuit board 180a on which the first connector 310' is formed.

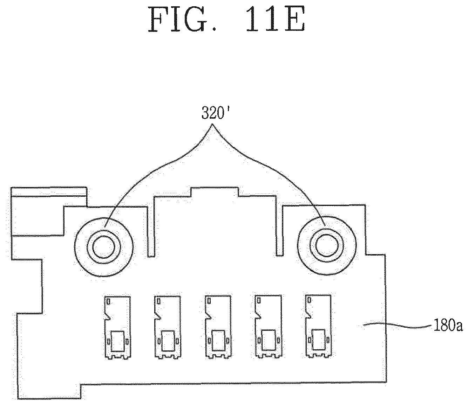

Meanwhile, FIGS. 11D and 11E are conceptual views illustrating an arrangement structure of the second electrode portion 320 arranged on a rear surface of the main body. The connector 320' for connecting with the first and second electrodes of the second electrode portion 320 is formed on the other surface of the main circuit board 180a. The connector 320' may have a shape protruded from the other surface of the main circuit board 180a. The second electrode portion 320 exposed to the rear surface of the main body is electrically connected with the main circuit board 180a by the protruded type connector 320'.

In accordance with this embodiment, the first and second electrode portions may electrically be connected to the main circuit board provided with the bio processor chip without a separate flexible circuit board or connection line. Therefore, since the distance for connecting the plurality of electrodes with the bio processor becomes short, impedance matching may be performed easily.

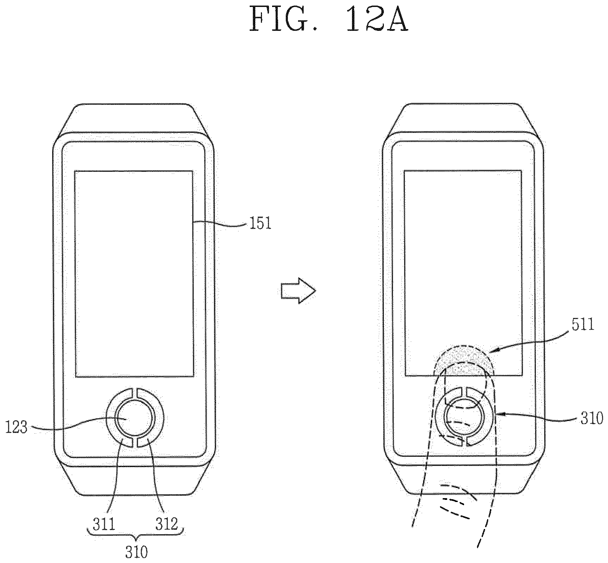

FIGS. 12A to 12D are conceptual views illustrating a control method for outputting touch guide information of a finger through a display unit.

The first electrode portion 310 according to these embodiments is arranged to adjoin the display unit 151 arranged on the main body 101. The first electrode portion 310 includes first and second electrodes 311 and 312.

The display unit 151 outputs first guide information 511 to allow all fingers of the user to touch the first and second electrodes 311 and 312. The first guide information 511 is formed to adjoin the edge area of the display unit 151 which is adjacent to the first electrode portion 310. The first guide information 511 may be comprised of an image corresponding to the tip of the finger.

For example, when the first guide information 511 may be comprised in a finger shape to be in contact with the display unit 151 when all fingers touch the first and second electrodes 311 and 312.

Therefore, if the user overlaps the finger with the first guide information 511, the finger may properly touch the first and second electrodes 311 and 312.

The controller 180 may control the display unit 151 to output the first guide information 511 if a body component measurement function is executed, if approach of the finger is sensed by a specific sensor (for example, proximity sensor), or if a touch which is not proper for the body component measurement is sensed in the first and second electrodes 311 and 312.

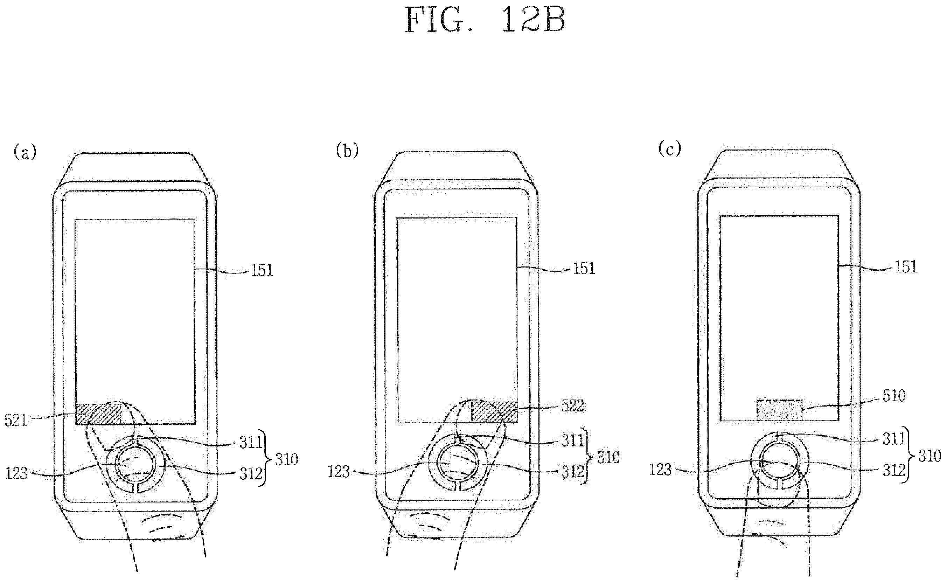

Referring to FIG. 12B, the display unit 151 outputs warning information on the basis of finger touch to the first and second electrodes 311 and 312. The user may identify whether the finger touch has been performed properly, by means of the warning information.

Referring to (a) and (b) of FIG. 12B, the display unit 151 senses a user's touch. The controller 180 senses a touch input applied to the finger of the user through the display unit 151, and if the touch input gets out of a predetermined proper touch area of the display unit 151, the controller 180 outputs first and second warning images 521 and 522.

The first and second warning images 521 and 522 may be displayed on a touch area where the finger is touched among areas other than the proper touch area.

Meanwhile, referring to (c) of FIG. 12B, the controller 180 controls the display unit 151 to output a third image 523 on the display unit 151 if the touch of the finger has been sensed in at least one area of the first and second electrodes 311 and 312 but the finger is touched at a specific range or less of the first electrode portion 310. In this case, the display unit 151 does not receive the finger's touch input.

According to this embodiment, the user may allow the finger to properly touch the first and second electrodes through the warning image.

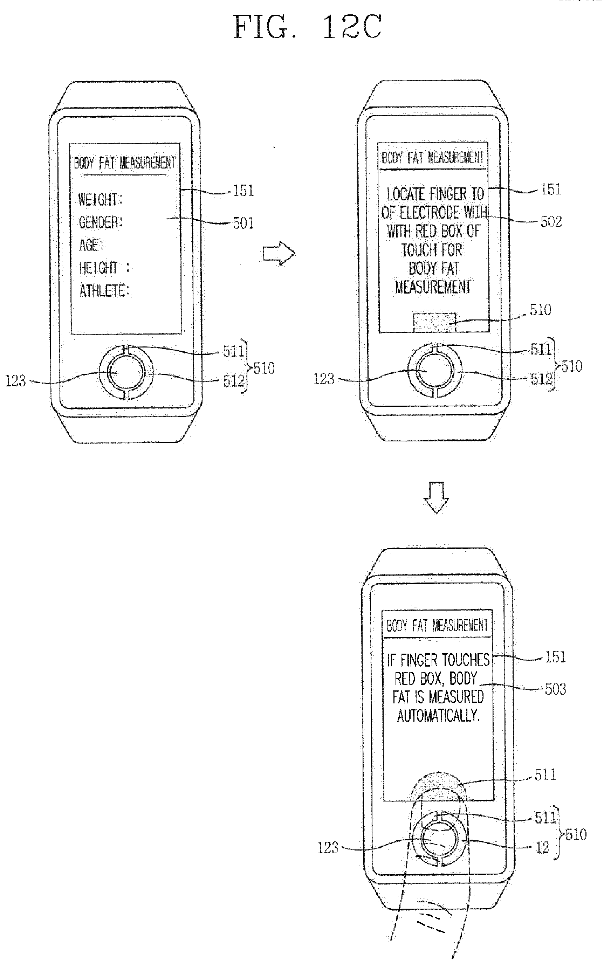

Referring to FIG. 12C, the display unit 151 outputs a body information screen 501 that includes a body state of the user. If there is no information required for the body information screen 501 or correction is required, the user may receive the required information from an external device or a specific server.

If the body component measurement function is executed, the first guide screen 502 and the guide image 510 are output. The guide image 510 is displayed on one area of the display unit 151, wherein the one area corresponds to an area where a part of the finger is touched if the finger properly touches the first and second electrodes 311 and 312. The first guide screen 502 may include description of a positon of the finger.

The controller 180 controls the display unit to change a second guide screen 503 to a third guide screen 504 and change the guide image 510 to the first guide information 511 if a touch is applied to at least one area of the first and second electrodes 311 and 312.

The second guide screen 502 may include information related to the positon of the finger and activation of the body component measurement function.

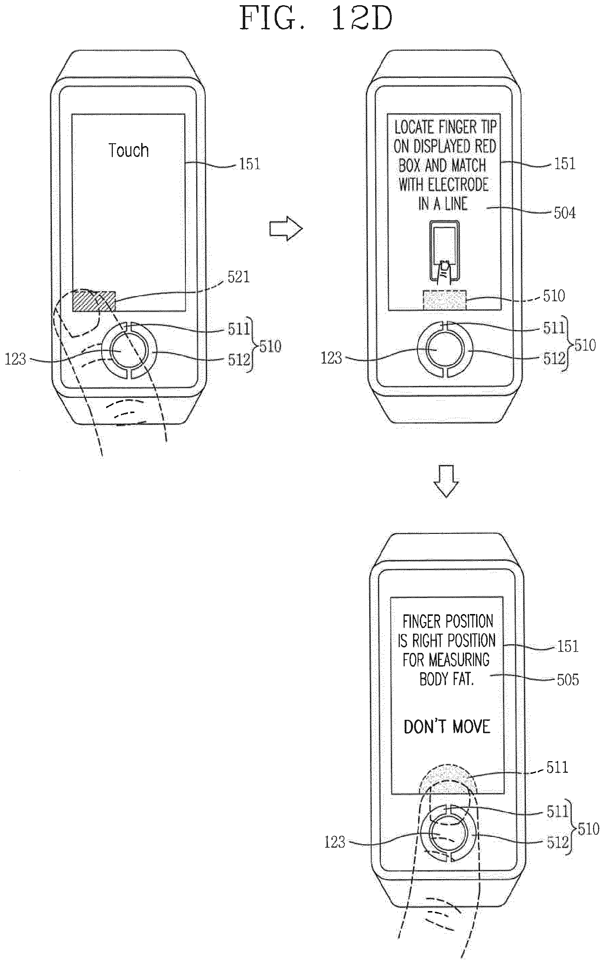

Referring to FIG. 12D, the display unit 151 may output a first warning image 521 in accordance with the positon of the finger, and may output the third guide screen 504 if a wrong position of the finger is maintained continuously. The third guide screen 504 may be output together with the guide image 510, and may include information for guiding the position of the finger and the first and second electrodes 311 and 312.

Meanwhile, the controller 180 may control the touch screen to output a fourth guide screen 505 if the finger exactly touches the first and second electrodes 311 and 312. The fourth guide screen 505 may include information for indicating 'don't move'.

According to these embodiments, the user may receive a guide for exact measurement through the display unit.

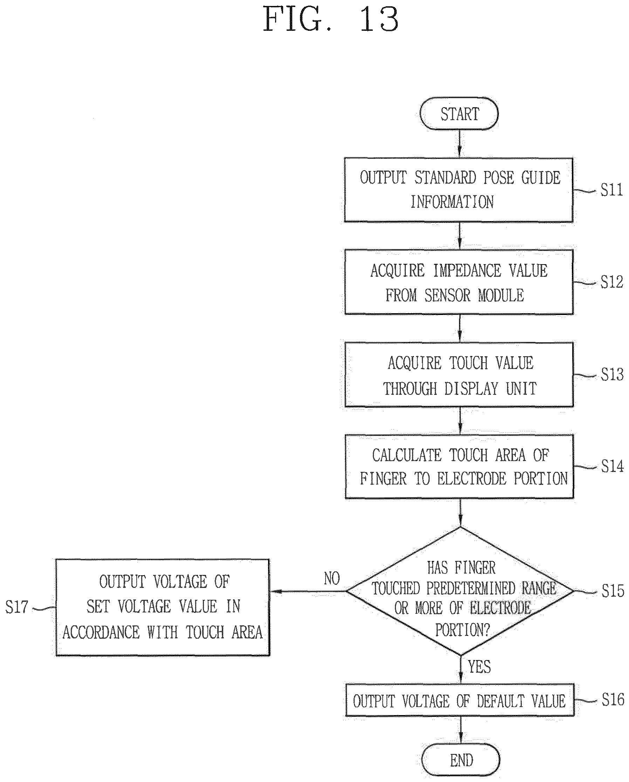

FIG. 13 is a flow chart illustrating a control method for controlling a body component measurement function on the basis of an area of a finger, which touches an electrode portion, in accordance with one embodiment.

Referring to FIG. 13, the display unit 151 outputs standard pose guide information (S11). The standard pose guide information may include image or text, and corresponds to screen information for guiding a pose of a finger when the finger touches the electrode portion.

An impedance value is acquired by the body component collecting sensor 200 (S12), and a touch value is acquired by a touch sensor provided in the display unit 151 while the impedance value is being acquired (S13). In this case, the touch value may correspond to a touch coordinate and a touch range of the touch input applied to the display unit 151.

The controller 180 calculates a touch area of the finger, which has touched the electrode portion, on the basis of the touch value (S14). The controller determines whether the finger has touched a predetermined range or more of the electrode portion, on the basis of the calculated touch area (S15).

The controller 180 outputs a voltage of a default value if it is determined that the finger has touched the predetermined range or more (S16). If the touch area of the finger, which has touched the electrode portion, is the predetermined range or more, the controller 180 sets a voltage value in accordance with the touched area and outputs the set voltage value (S17).

That is, the controller 180 controls the electrode portion to output a voltage greater than a default voltage value if it is determined that the finger has touched the electrode portion at a value less than the predetermined range. Therefore, the controller 180 may correct the impedance value by adjusting the voltage value even though the user allows the finger to again touch the electrode portion or does not move the finger.

The controller may guide the output of a proper resultant value by applying an alternating current voltage varied in accordance with the touch area without applying a fixed voltage value even though the area of the finger, which touches the electrode portion, is not sufficient.

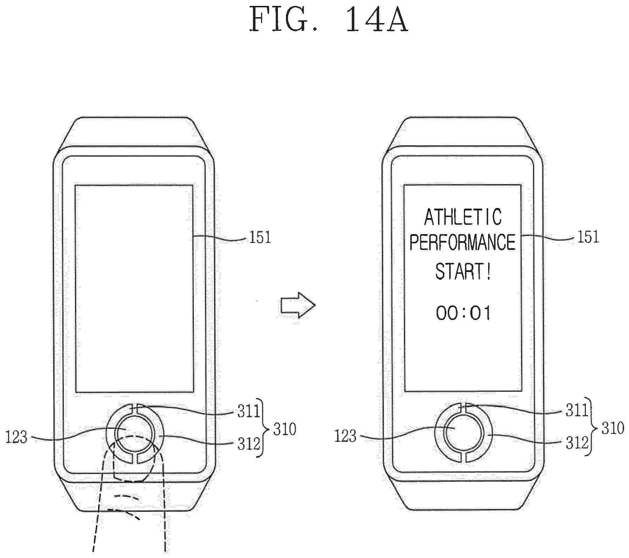

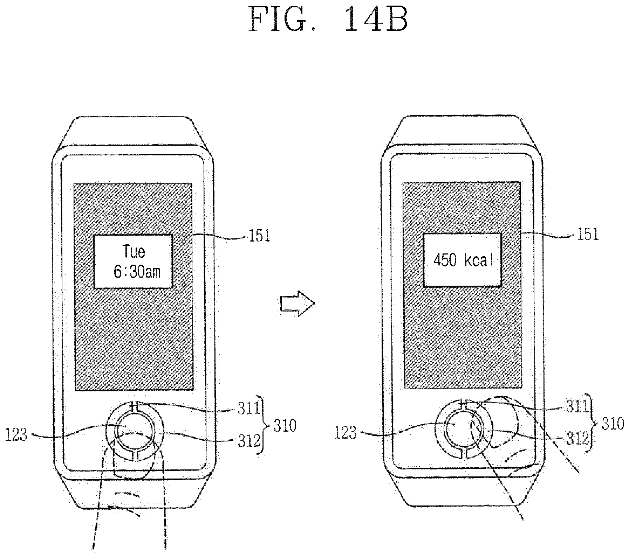

FIGS. 14A and 14B are conceptual views illustrating a control method for controlling a health band terminal by using an electrode portion.

The health band terminal 100 according to FIG. 14A includes an electrode portion 310 arranged to adjoin the user input unit 123. The controller 180 may control the health band terminal 100 through a control command formed by an external force applied to the user input unit 123. For example, the controller 180 may turn off the power source of the health band terminal 100 on the basis of a pressure of a specific time or more, which is applied to the user input unit 123.

Meanwhile, the controller 180 may activate an athletic performance mode if an external force is applied to the user input unit 123 for a time less than a specific time. If the athletic performance mode is activated, screen information related to the athletic performance mode may be output to the display unit 151.

Referring to FIG. 14B, the controller 180 switches a first screen (time) output onto the display unit 151 to a second screen (consumed calories) if the finger touches the first and second electrodes 311 and 312. Also, if the finger touches all or any one of the first and second electrodes 311 and 312, the controller 180 may switch the second screen to another screen.

If the finger touches any one of the first and second electrodes 311 and 312, the controller 180 outputs the output screens in an inverse order. That is, if the finger touches any one of the first and second electrodes 311 and 312 while the second screen is being output, the controller 180 controls the display unit 151 to switch the current screen to the first screen.

The controller 180 may determine whether the finger has touched all or any one of the first and second electrodes 311 and 312, on the basis of the change of the impedance value. As a result, the controller 180 may form different control commands. If the electrode portion 310 is arranged to adjoin the user input unit 123, the control according to the first and second electrodes 311 and 312 may correspond to the case that no pressure is applied to the user input unit 123.

However, the user input unit, which forms a control command by applying a pressure, may be omitted in the health band terminal 100 according to this embodiment. Therefore, the health band terminal may be controlled without a separate button type user input unit by using the electrode portion.

Although a control command for changing screen information is illustrated on the drawing as an example, the present invention is not limited to this control command.

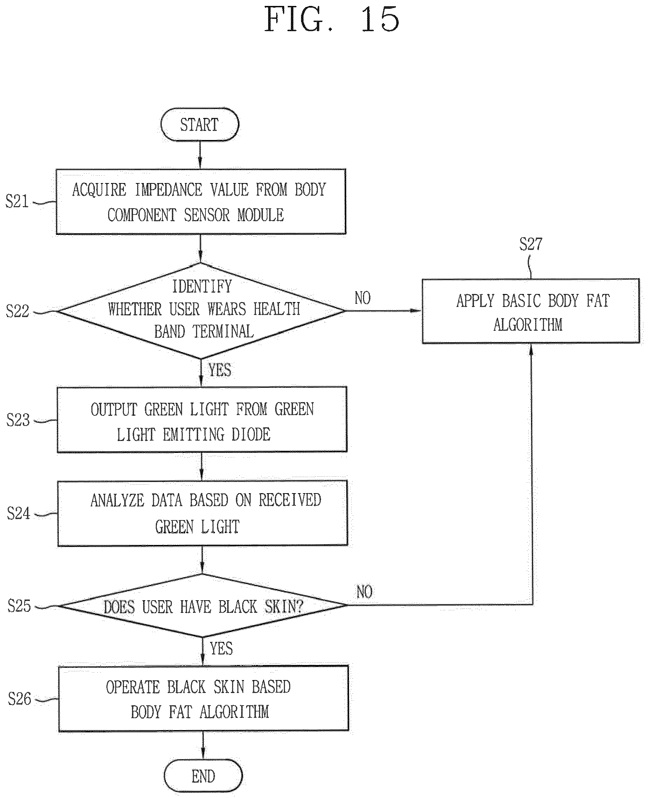

FIG. 15 is a flow chart illustrating a control method for applying different body fat algorithms on the basis of a skin color.

The controller 180 according to FIG. 15 identifies whether the user wears the health band terminal 100, on the basis of the IR sensor included in the PPG sensor unit 212 (S22). If it is not identified whether the user wears the health band terminal 100 or if it is sensed that the user does not wear the health band terminal 100, the controller 180 calculates body component information by applying the impedance value acquired by the electrode portion to a basic body fat algorithm (S27).

Meanwhile, if it is determined that the user wears the health band terminal 100, the controller 180 outputs green light from the green light emitting diode (S23). The controller 180 analyzes data through the received green light (S24) to identify whether the user has a black skin (S25). For example, the controller 180 may determine the black skin if intensity of the received light is a specific reference value or less.

If it is determined by the data that the user does not have a black skin, the controller 180 calculates body component information by using the basic body fat algorithm.

Meanwhile, if it is determined by the data that the user has a black skin, the controller 180 calculates body component information by using a black skin based body fat algorithm (S26).

According to this embodiment, body component information may be acquired more exactly by various algorithms through identification of different races whose body characteristics are different.

Various embodiments may be implemented using a machine-readable medium having instructions stored thereon for execution by a processor to perform various methods presented herein. Examples of possible machine-readable mediums include HDD(Hard Disk Drive), SSD(Solid State Disk), SDD(Silicon Disk Drive), ROM, RAM, CD-ROM, a magnetic tape, a floppy disk, an optical data storage device, the other types of storage mediums presented herein, and combinations thereof. If desired, the machine-readable medium may be realized in the form of a carrier wave (for example, a transmission over the Internet). The processor may include the controller 180 of the mobile terminal.

The foregoing embodiments and advantages are merely exemplary and are not to be considered as limiting the present disclosure. The present teachings can be readily applied to other types of apparatuses. This description is intended to be illustrative, and not to limit the scope of the claims. Many alternatives, modifications, and variations will be apparent to those skilled in the art. The features, structures, methods, and other characteristics of the exemplary embodiments described herein may be combined in various ways to obtain additional and/or alternative exemplary embodiments.

As the present features may be embodied in several forms without departing from the characteristics thereof, it should also be understood that the above-described embodiments are not limited by any of the details of the foregoing description, unless otherwise specified, but rather should be considered broadly within its scope as defined in the appended claims, and therefore all changes and modifications that fall within the metes and bounds of the claims, or equivalents of such metes and bounds are therefore intended to be embraced by the appended claims.

## Claims

1. A health band terminal comprising:
a main body (101) installable in one area of a body of a user;
a first electrode portion (310) arranged on one surface of the main body (101), having first and second electrodes (311, 312) arranged to adjoin each other;
a second electrode portion (320) arranged on the other surface of the main body (101); and
a controller (180) for calculating body component information by using an impedance value measured by the first and second electrode portions (310, 320),
wherein the second electrode portion (320) is protruded from the other surface of the main body (101) to touch the body of the user if the main body (101) is installed in the body of the user.

2. The health band terminal according to claim 1, wherein the second electrode portion (320) is arranged to adjoin a PPG sensor unit (212, 224) that includes a light receiving sensor and at least one light emitting diode, and the PPG sensor unit (212, 224) is formed on a first protrusion protruded from the other surface of the main body (101) at a predetermined thickness.

3. The health band terminal according to claim 2, wherein the second electrode portion (320) is formed on the first protrusion, and includes first and second electrodes (311, 312) spaced apart from each other by interposing the PPG sensor unit (212, 224) therebetween.

4. The health band terminal according to claim 2 or 3, further comprising a second protrusion arranged to adjoin the first protrusion, having the predetermined thickness, wherein at least one of the first and second electrode portions (310, 320) is formed on the second protrusion.

5. The health band terminal according to any one of claims 2 to 4, further comprising a dummy protrusion spaced apart from the second protrusion by interposing the first protrusion therebetween, wherein the first and second electrode portions (310, 320) are formed on the second protrusion.

6. The health band terminal according to one of claims 1 to 5, further comprising a user input unit arranged between the first and second electrodes (311, 312) of the first electrode portion (310) and configured to generate a control command by means of an external force.

7. The health band terminal according to claim 6, wherein the user input unit is arranged between the first and second electrodes (311, 312), extended toward the inside of the main body (101) and made of a nonconductor to insulate the first and second electrodes (311, 312) from each other.

8. The health band terminal according to claim 6 or 7, wherein the user input unit includes an actuator arranged inside the main body (101), to be pushed by movement of at least one of the first and second electrodes (311, 312).

9. The health band terminal according to any one of claims 1 to 8, further comprising a circuit board arranged inside the main body (101) and a connector connected with the circuit board, wherein, if the first electrode is arranged obliquely based on the circuit board, the first electrode is extended from the electrode portion and an end of a connection area which is in contact with the connector is made of an inclined plane.

10. The health band terminal according to any one of claims 1 to 9, further comprising a display unit (151) arranged on the one surface of the main body to adjoin the first electrode portion (310), sensing a touch input, wherein the controller (180) controls the display unit (151) to output guide information for guiding a position of a finger, which touches the first electrode portion (310).

11. The health band terminal according to claim 10, wherein the display unit (151) outputs a warning image if a touch is sensed in an area other than a predetermined area on the display unit (151) in a state that the finger of the user touches the electrode portion.

12. The health band terminal according to claim 10 or 11, wherein the controller (180) senses a touch range of the touch input applied to the display unit (151) and adjusts a voltage value which will be output through the first and second electrode portions (310, 320) on the basis of the touch range.

13. The health band terminal according to any one of claims 1 to 12, wherein the controller (180) forms different control commands based on that the finger of the user touches at least one of the first and second electrodes (311, 312) of the first electrode portion (310).

14. The health band terminal according to claim 13, further comprising a user input unit adjacent to the first and second electrodes (311, 312) and provided with a control command formed by an external force, wherein the controller (180) forms the different control commands on the basis of the touch of the finger to the first and second electrodes (311, 312) if the external force is not sensed by the user input unit.

15. The health band terminal according to any one of claims 1 to 14, further comprising a PPG sensor unit (212, 224) installed in the main body, including a light receiving sensor and a light emitting diode, a skin color is sensed based on intensity of light received by the light receiving sensor, and the controller (180) calculates the body component information by using a body fat algorithm based on the skin color.
